# EUROPÄISCHE PATENTANMELDUNG

(11) **EP 1 464 338 A1**
(43) Veröffentlichungstag der Anmeldung: **06.10.2004**
(21) Anmeldenummer: 04004594.0
(22) Anmeldetag: 27.02.2004
(51) Int. Cl.: A61K 41/00, A61K 49/00, A61K 9/00

(54) **Teilchen mit einer Membran**

(30) Priorität: 04.04.2003 DE 10315538
(71) Anmelder: SIEMENS AKTIENGESELLSCHAFT, 80333 München (DE)
(72) Erfinder: Geiger, Wolfgang, Dr., 91522 Ansbach (DE)

(57) **Zusammenfassung**

In einer Fluidbahn, insbesondere einer Blutbahn, transportierbares Teilchen mit einer einen Partikelkern (2) einschließenden Membran (3) mit einer Anzahl in einer Matrix (4) eingebundener Funktionselemente (5,5a,5b,8,10,16), welche in Abhängigkeit von der Konzentration eines Körperstoffes (7) einen Stofftransport durch die und/oder eine Stoffanlagerung an der Membran (3) bewirken.

## Beschreibung

Die Erfindung betrifft ein in einer Fluidbahn, insbesondere einer Blutbahn eines Menschen oder Tieres, transportierbares Teilchen, vorzugsweise für analytische, diagnostische und/oder therapeutische Anwendungen. Ein derartiges Teilchen ist beispielsweise aus der DE 691 26 535 T2 bekannt.

Aus der US 4,329,332 A sind Teilchen mit einem Durchmesser von weniger als 1 µm bekannt, welche aus einem polymerisierten Material gebildet sind und eine biologisch aktive Substanz enthalten. Informationen zu solchen medizinisch einsetzbaren, auch als Nanokapseln bezeichneten Partikeln enthält auch die Dissertation "NMR-Untersuchungen an Nanokapsel-Dispersionen" (Dirk Hoffmann, Universität Duisburg, Chemie, 4.9.2000). Als Nanokapseln werden hierin sphärische, hohle Objekte bezeichnet, die in der Medizin als gewebespezifische Wirkstoff-Träger-Systeme eingesetzt werden sollen. Ein in der Nanokapsel eingeschlossener Wirkstoff ist von einem Polymergerüst umgeben. Ziel ist es, damit ein "Drug-Targeting-System" zu schaffen, welches den Wirkstoff umschließt und ihn geschützt zum Zielgewebe trägt, wo er seine heilende Wirkung entfalten kann, ohne den restlichen Körper zu schädigen. Der Durchmesser der Wirkstoff-Träger-Systeme sollte bei intravenöser Applikation kleiner als 4 µm sein, damit sie durch die kleinsten Blutgefäße im Körper wandern und keine für den Patienten lebensbedrohlichen Embolien auslösen können. Ein Wirkstoff-Träger-System kann beispielsweise in Form eines Vesikels eine kugelig in sich abgeschlossene Membranlamelle aufweisen, die aus einer Lipid-Doppelschicht aufgebaut ist. Liposome als künstlich hergestellte Vesikel haben typischerweise einen Durchmesser zwischen 20 nm und 3 µm sowie eine Membran mit einer Dicke von ca. 5 nm. Pharmazeutische Anwendungen von Liposomen beruhen insbesondere auf der Möglichkeit, hydrophile Moleküle in deren wässrigem Innenraum zu verkapseln. Durch diese Verkapselung dient das Liposom als Permeationsbarriere mit Depoteffekt.

Alternativ zu Liposomen können als Wirkstoff-Träger-Systeme, insbesondere aus physiologischen Lipiden oder aus Lipiden aus physiologischen Komponenten, auch feste Lipidnanopartikel, auch als SLN (Solid-Lipid-Nanoparticles) bezeichnet, hergestellt werden. Um bei derartigen festen Lipidnanopartikeln einen "Targeting-Effekt" zu erreichen, wird die Nanokapselwand mit einer stabilisierenden Tensidschicht umgeben. Eine gezielte Freisetzung des Wirkstoffs am gewünschten Ort ist auf diese Weise jedoch nur eingeschränkt möglich.

Biologisch wirksame Mikrokapseln, die durch Einschließen von biologischen Zellen in einer Umhüllung aus biokompatiblem Polymermaterial hergestellt sind, sind beispielsweise in der DE 102 03 628 A1 beschrieben. Diese Mikrokapseln enthalten fusionierte biologische Zellen, wobei eine Fusion im elektrischen Feld, das heißt eine Elektrofusion, durchführbar ist, die den Vorteil haben soll, dass die Zahl der zu fusionierenden Zellen einstellbar ist. Die Fusion wird vorzugsweise vor der Verkapselung, das heißt vor dem Einschließen der Zellen in das Polymermaterial, durchgeführt.

Mikroteilchen oder Nanoteilchen, insbesondere für kosmetische oder pharmazeutische Zusammensetzungen, sind auch aus der DE 199 32 216 A1 bekannt. Es ist die Möglichkeit beschrieben, mittels poröser Teilchen Moleküle aus einem flüssigen Medium zu entfernen, zu "ernten", oder eingeschlossene Moleküle in langsamer Weise an einer Wirkstelle freizusetzen. Eine Langzeitfreisetzung oder verzögerte Freisetzung eines eingeschlossenen Moleküls kann unter anderem durch die pH-Wert-Abhängigkeit der Löslichkeit bestimmter Verbindungen beeinflusst werden.

In der DE 693 29 295 T2 sind Polymermikrosphären mit einem Durchmesser von weniger als 180 µm zur kontrollierten Freisetzung von Wachstumshormonen beschrieben. Ein Langzeit- oder verlängertes Freisetzen eines Arzneimittels soll, beeinflusst unter anderem durch die Art einer oder mehrerer Polymer-Zusammensetzungen, kontinuierlich oder diskontinuierlich, linear oder nichtlinear erfolgen.

In der DE 691 26 535 T2 beschriebene biokompatible, zur Transplantation in ein Tier vorgesehene Mikrokapseln weisen eine Membran von mehr als einer Schicht auf. Hierbei ist die äußerste, polykationische Schicht der Membran ionisch mit einer weiteren Membranschicht vernetzt und aus einem wasserlöslichen nichtionionischen Polymer aufgebaut. Die Mikrokapseln sollen eine gegen Zelladhäsion widerstandsfähige Oberfläche haben und Zellen enthalten, die in der Lage sind, Nährstoffe und Signalmoleküle zu empfangen und ein gewünschtes Produkt herzustellen.

Aus der DE 37 82 840 T2 ist ein Arzneimittel in Form einer Mikrokapsel mit verzögerter Freigabe bekannt, dessen Hülle beispielsweise unter anderem aus Zelluloseazetetphtalat gebildet ist. Die Auflösung der Mikrokapsel, welche einen feinkörnigen Kern eines wasserlöslichen Medikaments einschließt, ist durch Enzyme beeinflussbar.

Der Erfindung liegt die Aufgabe zugrunde, ein in einer Fluidbahn, insbesondere einer Blutbahn eines Menschen oder Tieres, transportierbares Teilchen anzugeben, dessen Wirkung sich besonders gezielt am gewünschten Ort, insbesondere im gewünschten Gewebe, entfaltet. Des Weiteren ist es Aufgabe der Erfindung, ein analytisches und/oder diagnostisches Verfahren unter Nutzung eines solchen Teilchens anzugeben.

Diese Aufgabe wird erfindungsgemäß gelöst durch ein Teilchen mit den Merkmalen des Anspruchs 1 sowie durch ein Verfahren mit den Merkmalen des Anspruchs 15. Das in einer Fluidbahn, vorzugsweise einer Blutbahn eines Menschen oder Tieres, transportierbare Teilchen weist eine einen Partikelkern einschließende Membran mit einer Anzahl in einer Matrix eingebundener Funktionselemente auf, welche in Abhängigkeit von der Konzentration eines Körperstoffes einen Stofftransport durch die und/oder eine Stoffanlagerung an der Membran bewirken. Ein Stofftransport durch die Membran kann hierbei nach innen und/oder nach außen erfolgen. Funktionselemente sind insbesondere Schleusenelemente, beispielsweise Ionenkanäle, und/oder Detektoren, insbesondere zur Detektion des in einem das Teilchen umgebenden Medium enthaltenen Körperstoffes. Die Matrix hat insbesondere die Aufgabe, das Funktionselement am Partikelkern zu fixieren und/oder den Partikelkern, gegebenenfalls zusammen mit dem bzw. den Funktionselementen, nach außen abzuschließen. Der Partikelkern kann beispielsweise mit einem Pharmakon gefüllt sein und/oder einen Hohlraum zur Aufnahme eines Stoffes aus dem das Teilchen umgebenden Medium aufweisen.

Das Teilchen ist nach einer bevorzugten Ausgestaltung zur Anwendung in einem diagnostischen Verfahren vorgesehen. Hierbei wird in Abhängigkeit von der Konzentration eines Körperstoffes in dem das Teilchen umgebenden Medium ein körpereigener Stoff an der Membran angelagert, in die Membran eingebaut, und/oder durch die Membran hindurch in den Partikelkern eingeschleust. Allgemein ist die Funktionalität eines internalisierten Rezeptors, Kanals oder einer Austauschmembran gegeben. Hinsichtlich der prinzipiellen Funktionsweise von Rezeptoren wird beispielhaft auf Thyrosinkinaserezeptoren verwiesen. Der im oder am Teilchen akkumulierte körpereigene Stoff ist nicht notwendigerweise mit dem genannten Körperstoff, dessen Konzentration den Anlagerungs- oder Einlagerungsvorgang beeinflusst, identisch. Das Teilchen ist besonders geeignet, einen in niedriger Konzentration vorhandenen Stoff einer Flüssigkeit, z.B. Blut, Serum, Plasma, Harn, Sputum, Liquor oder einer sonstigen tierischen oder humanen Körperflüssigkeit selektiv zu sammeln. Ebenso kann das Teilchen jedoch auch genutzt werden, um in Lösungen, Wässern, Abwässern, Extrakten oder sonstigen flüssigen Analysenproben, z.B.

Getränken, selektiv einen oder mehrere Stoffe einzusammeln. Der in dem das Teilchen umgebenden nicht notwendigerweise fließenden Medium enthaltene Stoff, dessen Konzentration die Eigenschaften der Membran beeinflusst, wird unabhängig von der Art des Mediums einheitlich als Körperstoff bezeichnet.

Durch das selektive Einsammeln des humanen oder sonstigen Stoffes wird ein sonst erforderlicher Anreicherungs- und Konzentrationsschritt ersetzt. Während beispielsweise bei einer Blutuntersuchung die quantitative Bestimmung eines hoch verdünnten Stoffes nach dem Stand der Technik nur mit einer Blutprobe von relativ hohem Volumen möglich wäre, ist durch die Anreicherung des zu untersuchenden Stoffes an den hierzu in einem diagnostischen Verfahren eingesetzten Teilchen zumindest eine gravierende Reduzierung des Probenvolumens möglich.

Beim Einsatz des Teilchen zur Akkumulation eines körpereigenen Stoffes wird dieser nicht notwendigerweise in unveränderter Form im Teilchen eingelagert oder an diesem angelagert. In jedem Fall wird das Teilchen jedoch detektierbar verändert. Die Detektion des beispielsweise in einer Blutbahn transportierten Teilchens erfolgt bevorzugt ohne physikalische Probenahme, d.h. beispielsweise Blutentnahme. Das Teilchen ist hierzu zumindest in dessen Form nach der Akkumulation des körpereigenen Stoffes mit einem bildgebenden Verfahren darstellbar. Als medizintechnische Verfahren kommen hierbei insbesondere Ultraschallverfahren, NMR-Verfahren, CT-Untersuchungen (Computer-Tomographie), Fluoreszenz-Messverfahren sowie die Protonen-Emissions-Tomographie in Betracht. Durch die Kopplung der Anwendung des Teilchens mit solchen bildgebenden medizintechnischen Verfahren ist insgesamt ein elektronisches diagnostisches Konzept realisierbar. Die IT-Komponente wird auch als In-Vivo-Logistik-Intelligenz (Transportomics) bezeichnet. Diese kann, wie nachfolgend noch näher erläutert wird, auch in ein therapeutisches Konzept, welches die von außen, d.h. extrakorporal, beeinflussbaren Eigenschaften des Teilchens nutzt, integriert sein.

Das Teilchen, je nach Dimensionierung auch als Nanopartikel bezeichnet, kann zusätzlich oder alternativ zur Akkumulation eines körpereigenen Stoffes auch zur Abgabe eines Stoffes, insbesondere eines Pharmakons, vorgesehen sein. Die Freisetzungsrate des Pharmakons ist dabei von der Konzentration eines Körperstoffes im das Teilchen umgebenden Medium abhängig. Die Abgabe des Pharmakons kann beispielsweise dadurch erfolgen, dass dieses durch ein als Schleusenelement fungierendes Funktionselement innerhalb der Matrix hindurchgeschleust wird, oder durch vollständige Auflösung der Membran unter gleichzeitiger Freigabe des das Pharmakon enthaltenden oder mit dem Pharmakon identischen Partikelkerns. Im Fall der Auflösung der Membran in Abhängigkeit von der Konzentration oder Anwesenheit des Körperstoffes wirkt die Membran als Ganzes als Funktionselement zur Freisetzung des Pharmakons.

Das Pharmakon kann nicht nur im Partikelkern, sondern stattdessen oder zusätzlich auch in der Membran enthalten sein. Die Membran kann hierbei, wie in allen anderen Ausführungsbeispielen auch, einlagig oder mehrlagig aufgebaut sein. Im Fall einer mehrlagigen Membran, in welcher ein Pharmakon eingebunden ist, befindet sich dieses bevorzugt in der inneren Schicht bzw. den inneren Schichten der Membran. Bei der diagnostischen oder therapeutischen Verwendung des Teilchens ist insbesondere dessen Biokompatibilität und Bioabbaubarkeit relevant. Die biologische Halbwertzeit des Teilchens sollte lang genug sein, um bei diagnostischer Anwendung einen ausreichenden Akkumulationseffekt zu erzielen und bei therapeutischen Anwendungen eine gegebenenfalls den Patienten belastende zu häufige Applikation des Medikaments zu vermeiden. Um beiden Aspekten in ausreichendem Maße Rechnung zu tragen, ist bevorzugt eine mittel- bis langfristige enzymatische Angreifbarkeit der Membran im humanen oder tierischen Körper vorgesehen, d.h. ein Abbau des Teilchens innerhalb der Dauer von mindestens einigen Stunden, vorzugsweise einigen Tagen oder Wochen. Der Partikelkern ist im Allgemeinen schneller, beispielsweise durch Metabolisierung, abbaubar als die Membran.

Der Partikelkern umfasst oder bildet nach einer bevorzugten Weiterbildung einen zur Stoffumwandlung vorgesehenen Reaktionsbereich. Dies gilt sowohl bei der Stoffaufnahme als auch bei der Stoffabgabe in den bzw. aus dem Partikelkern. Im letztgenannten Fall enthält der Partikelkern nicht das Pharmakon in dessen anzuwendender Form, sondern lediglich mindestens ein Vorprodukt. Die Umwandlung dieses Vorprodukts zum Pharmakon wird durch ein auf das Teilchen einwirkendes Signal ausgelöst. Dieses Signal kann durch eine Körperstoffkonzentration im das Teilchen umgebenden Medium gegeben sein. Allgemein wird durch ein biochemisches Signal oder eine biochemische Reaktion ein Vorprodukt aus dem Partikel freigesetzt und durch lokale Enzyme in das gewünschte Produkt umgewandelt. Eine enzymatische Reaktion bewirkt somit eine Prodrug-Drug-Umwandlung.

Die Aktivität des Teilchen ist durch ein extrakorporales Signal beeinflussbar. Ein solches extrakorporales Signal kann in Form einer physikalischen Kraft oder eines Feldes, beispielsweise einer Ultraschalleinstrahlung, oder eines Magnetfeldes gegeben sein. Im Fall einer dermatologischen Anwendung des Teilchens ist auch eine Aktivierung, d.h. gezielte Umwandlung, Stoffabgabe und/oder -aufnahme, durch Licht, insbesondere Licht in einem bestimmten begrenzten Wellenlängenbereich, beispielsweise Infrarotlicht, realisierbar. Die Membran weist hierzu einen auf die entsprechende elektromagnetische Strahlung reagierenden Rezeptor auf, welcher die Durchlässigkeit der Membran und/oder eine Stoffumwandlung im Partikelkern beeinflusst. Der Rezeptor kann als Funktionselement innerhalb der Membran in eine Matrix eingebunden sein oder selbst die gesamte Membran bilden.

Durch die Steuerbarkeit der Aktivität des Teilchens von außen ist die Möglichkeit gegeben, statt eines Arzneistoffes nur noch die Information in den Körper einzubringen, welche festlegt, welcher Stoff zu welchem Zeitpunkt wo im Körper produziert wird. Solange diese Information, die beispielsweise über ein Ultraschallsignal, IR-Signal oder über ein Magnetfeld in ein Körper eingespeist wird, nicht übermittelt wird, kann das Partikel derart beschaffen sein, dass sich dieses passiv, d.h. biologisch neutral verhält oder lediglich eine beschränkte Grundaktivität entfaltet. Eine Therapie ist somit mittels eines sogenannten IT Pathway Logistikmoduls von außen kontrollierbar und auch mit diagnostischen Verfahren koppelbar, wodurch insgesamt eine geschlossene Regelung (closed loop) realisierbar ist. Das von dem Teilchen freizusetzende Arzneimittel ist mittels eines sogenannten In Vivo Transportsystems individualisiert verabreichbar, d.h. die therapeutische Anwendung ist, ebenso wie die diagnostische Anwendung personalisierbar.

Eine besonders vorteilhafte Anwendung des Teilchens ist erzielbar, wenn dessen Reaktionsbereich, der einen Teil des Partikelkerns bildet oder mit diesem identisch ist, zur Umwandlung eines körpereigenen, d.h. humanen oder tierischen, Zwischenstoffes vorgesehen ist. Diese Anwendung kommt in Betracht, wenn der humane bzw. tierische Organismus, nicht mehr in der Lage ist, aus dem Zwischenstoff einen benötigten Endstoff selbst zu produzieren. Sofern die Produktionsrate des Zwischenstoffes im Organismus vom Bedarf am letztlich benötigten Endstoff abhängig ist, ergibt sich mit der Umwandlung des Zwischenstoffes zum Endstoff im Teilchen eine selbstregulierende Endstoffproduktion. Sowohl die Menge des vom Teilchen ausgestoßenen Endstoffes als auch die örtliche Verteilung des Endstoffes sind durch die Kopplung der Reaktion an den körpereigenen Zwischenstoff dem tatsächlichen Bedarf angepasst.

Die Dimensionierung des Teilchens wird entsprechend den jeweiligen Erfordernissen innerhalb eines weiten Bereichs gewählt. Bevorzugt beträgt der Außendurchmesser zwischen 50 nm und 10 µm, insbesondere zwischen 200 nm und 2 µm, sowie die Stärke der Membran zwischen 2 nm und 1 µm, insbesondere maximal 100 nm. Als Material der Membran, insbesondere der die Funktionselemente einbettenden Matrix, wird bevorzugt ein Polymer, insbesondere ein biologisch abbaubares Polymer gewählt. In dieses Polymer können in Form einer Polymerelektronik elektronische Komponenten eingbaut sein, insbesondere zur bidirektionalen datentechnischen Verbindung mit externen IT-Komponenten. Eine solche Polymerelektronikmembran bildet bevorzugt die Schnittstelle zu bildgebenden medizintechnischen Verfahren.

Nachfolgend wird ein Ausführungsbeispiel der Erfindung anhand einer Zeichnung näher erläutert. Hierin zeigen jeweils in stark vereinfachter schematischer Darstellung:
- Fig. 1: ein Teilchen mit einem internalisierten körpereigenen Stoff,
- Fig. 2: ein Teilchen mit einem an eine Membran angebundenen körpereigenen Stoff,
- Fig. 3: ein Teilchen mit einem aus diesem freisetzbaren Pharmakon,
- Fig. 4: ein Teilchen mit einem Reaktionsbereich innerhalb eines Partikelkerns, und
- Fig. 5: ein Teilchen mit einem in Abhängigkeit von einem extrakorporalen Signal freisetzbaren Pharmakon.

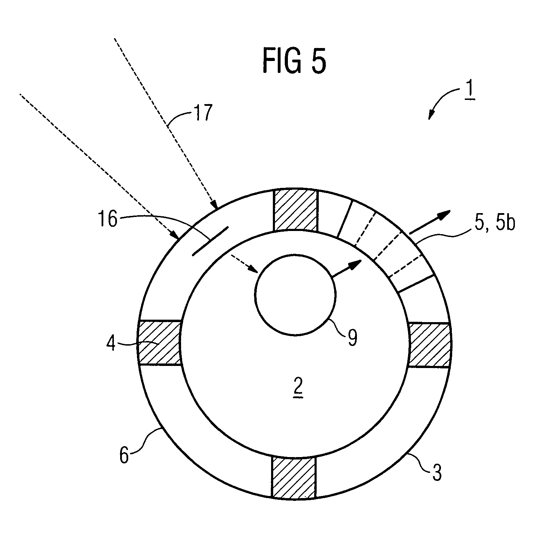

Einander entsprechende Teile bzw. Parameter sind in allen Figuren mit den gleichen Bezugszeichen gekennzeichnet. Die Gesamtheit der Merkmale des Teilchens nach Anspruch 14 ist in Fig. 5 symbolhaft dargestellt. Anhand der übrigen Figuren werden Überlegungen im Zusammenhang mit der Erfindung erläutert.

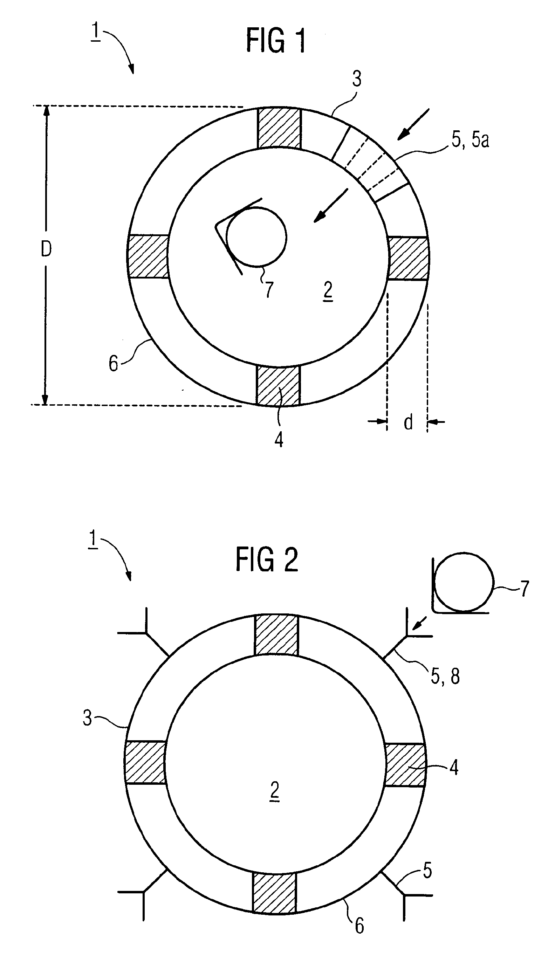

Die Fig. 1 bis 5 zeigen in verschiedenen Ausführungsformen jeweils ein in einer Blutbahn eines Menschen transportierbares Teilchen 1. Sämtliche anhand der Fig. 1 bis 5 erläuterten Funktionen sind in beliebiger Weise auch in einem einzigen Teilchen 1 kombinierbar. Das Teilchen 1 setzt sich grundsätzlich zusammen aus einem Partikelkern 2 und einer diesen umgebenden Membran 3. Die Membran 3 weist als Gerüst eine Matrix 4 auf, in welche nachstehend noch näher erläuterte Funktionselemente 5 eingebunden sein können. Unter Einbindung wird hierbei auch die äußere Anlagerung eines Funktionselementes 5 (Fig. 2) verstanden. Im Fall der äußeren Anlagerung eines oder mehrerer Funktionselemente 5 kann die Membran 3 auch vollständig aus der Matrix 4 gebildet sein. In den Ausführungsbeispielen nach den Fig. 1 bis 5 sind in die Matrix 4 jeweils mehrere Membranelemente 6 eingebunden, in die wiederum teilweise Funktionselemente 5 eingebettet sind. Ebenso können jedoch auch Funktionselemente 5 unmittelbar in die Matrix 4 eingebunden sein. Des Weiteren kann die Membran 3 auch ausschließlich aus einem oder mehreren Funktionselementen 5 gebildet sein.

Die Membran 3 ist bevorzugt vollständig oder überwiegend aus einer Polymerschicht, insbesondere aus Polylaktiden, Silikaten, Kautschuk oder Kunststoff, gebildet. Ebenso kann die Membran 3 aus biologischem und/oder synthetischem Gewebe aufgebaut sein. Im Fall eines Aufbaus aus körpereigenen humanen Zellen können diese jeweils einzelne Membranelemente 6 bilden. Die zur Herstellung der Membran 3 genutzten Zellen werden lysiert und durch Kittpartikel, wie Ligasen, Klebstoffe oder Mikroorganismen unter Bildung der Matrix 4 miteinander verbunden. Zwischen einzelnen, jeweils ein Membranelement 6 bildenden Zellmembranstücken können, vergleichbar wie bei Darmzellen, so genannte tight junctions als Funktionselemente 5 gebildet sein. Die Teilchen 1 können in humanmedizinischen Anwendungen auf jede beliebige Weise, beispielsweise oral, nasal, transdermal, pulmonal oder peritonal appliziert werden. Der im Weiteren noch näher erläuterte Transport von Stoffen durch die Membran 3 oder eine Stoffanlagerung an der Membran 3 kann durch jegliche im mikroskopischen Bereich nutzbare Kräfte erfolgen. Insbesondere zu nennen sind hierbei elektroosmotische Kräfte, eine elektrostatische Bindung und anschließende Polarisierung (Richtungswechsel), biologische Potentialänderungen (Synapsen, Ionenkanäle), sowie promotorinduzierte oder durch ein Transportergen induzierte Vorgänge (vgl. Reportergenassays). Ebenso ist ein mechanisches Swelling (push/pull) oder ein Port/Antiport-Mechanismus nutzbar. Das Teilchen 1 ist durch externe Kräfte oder Felder, d.h. Signale, die außerhalb des zu behandelnden oder zu beobachtenden menschlichen oder tierischen Körpers erzeugt sind, beeinflussbar (Fig. 5).

Im Folgenden wird auf die in den Figuren dargestellten Ausführungsbeispiele im Einzelnen näher eingegangen. Die Fig. 1 und 2 zeigen jeweils ein Teilchen 1, welches zum Sammeln oder Akkumulieren eines körpereigenen Stoffes 7 vorgesehen sind. Das auch als Nanopartikel bezeichnete Teilchen 1, welches in der Blutbahn eines Patienten transportierbar ist, ist in beiden Fällen für diagnostische Zwecke vorgesehen. Der Partikelkern 2 ist in beiden Fällen zunächst leer oder mit einer beispielsweise wässrigen Lösung gefüllt. Das Teilchen 1, das nicht notwendigerweise kugelförmige Gestalt hat, hat einen Außendurchmesser D von beispielsweise 3 µm. Die Membran 3 weist eine Stärke d von beispielsweise 5 nm auf. Im Ausführungsbeispiel nach Fig. 1 ist in die Membran 3 ein als Schleusenelement zu bezeichnendes Funktionselement 5 eingebettet, dessen Dicke nicht notwendigerweise der Dicke d der übrigen Membran 3 entspricht. Das Funktionselement 5 kann zumindest zu einem Anteil auch Bestandteil des Partikelkerns 2 sein. Beispielhaft ist in Fig. 1 lediglich ein einziges Schleusenelement 5 dargestellt; in ähnlicher Weise können jedoch auch mehrere Schleusenelemente 5 in die Membran 3 eingebunden sein oder diese bilden. Das Schleusenelement 5 ist von außen nach innen selektiv durchlässig für den körpereigenen Stoff 7. Der körpereigene Stoff 7 wird somit im Laufe der Anwendung des Teilchens 2 im Partikelkern 2 gesammelt. Die somit erhaltene hohe Konzentration des körpereigenen Stoffes 7 innerhalb des Teilchens 1 erleichtert die Detektion des körpereigenen Stoffes 7 wesentlich. Insbesondere ist es durch diese Akkumulation des körpereigenen Stoffes 7 möglich, diesen, wenn er beispielsweise Eisen enthält, mittels an sich bekannter medizintechnischer diagnostischer Verfahren wie magnetischer Resonanzverfahren (NMR) zu detektieren, obwohl der körpereigene Stoff 7 in derart geringer Konzentration beispielsweise im Blut des Patienten vorhanden ist, dass ein solches bildgebendes Verfahren ansonsten nicht anwendbar wäre. Die Lebensdauer der mit dem körpereigenen Stoff 7 beladenen Teilchen 1 im humanen bzw. tierischen Körper sollte in der Regel begrenzt sein. Zu diesem Zweck ist die Membran 3 aus Substanzen aufgebaut, welche im Körper enzymatisch angreifbar sind.

Das Ausführungsbeispiel nach Fig. 2 unterscheidet sich vom Ausführungsbeispiel nach Fig. 1 dadurch, dass der körpereigene Stoff 7 außerhalb des Teilchens 1 angelagert wird. Der Partikelkern 2 kann in diesem Ausführungsbeispiel entfallen. Zur Anlagerung des körpereigenen Stoffes 7 sind Kopplungselemente 8 vorgesehen, welche zugleich die Funktion von auf den körpereigenen Stoff 7 ansprechenden Detektoren haben. Durch die Anlagerung des körpereigenen Stoffes 7 am Teilchen 1 wächst dessen Durchmesser D nur in derart unbedeutendem Maße, dass die Transportierbarkeit des Teilchens 1 praktisch nicht beeinflusst ist. Sämtliche Darstellungen sind nicht maßstäblich.

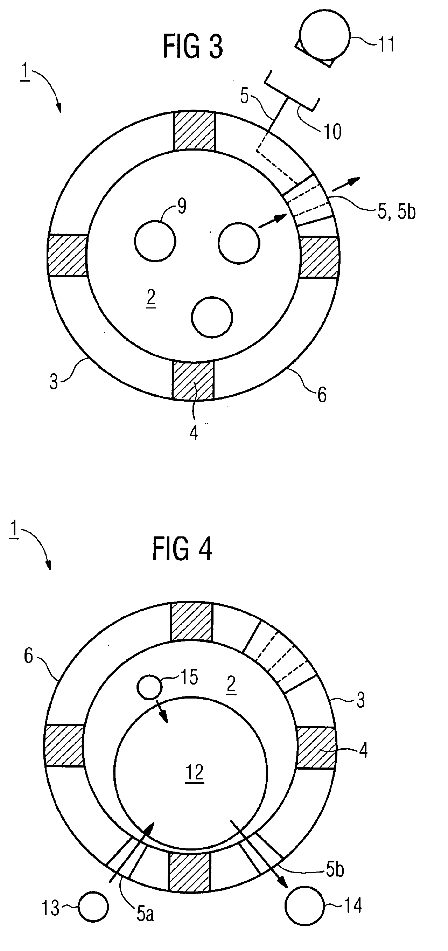

Im Ausführungsbeispiel nach Fig. 3 ist im Partikelkern 2 ein Pharmakon 9 enthalten, welches durch ein Schleusenelement 5 als Funktionselement hindurch aus den Teilchen 1 herausgeschleust werden kann. Das Schleusenelement 5 wirkt, durch eine gestrichelte Linie angedeutet, mit einem Detektorelement 10 als weiterem Funktionselement 5 zusammen. Das Detektorelement 10 ist im Gegensatz zu dem in Fig. 2 dargestellten Kopplungselement 8 jedoch primär nicht zur Stoffanlagerung vorgesehen. Aufgabe des Detektorelements 10 ist es lediglich, die Anwesenheit eines Körperstoffes 11 festzustellen und infolgedessen die Durchlässigkeit des Schleusenelementes 5 für das Pharmakon 9 herzustellen. Der Körperstoff 11 bleibt bei diesem Vorgang nicht notwendigerweise am Detektorelement 10 angelagert. Beispielsweise kann das Detektorelement 10 ein Kaliumrezeptor sein, welcher Kalium als Körperstoff 11 aufnimmt. Mit dem Kaliumrezeptor 10 ist das auch als Transportrezeptor bezeichnete Schleusenelement 5 derart gekoppelt, dass das Pharmakon 9 freigesetzt wird. Auf diese Weise wird das Pharmakon 9 gezielt kaliumreichem Gewebe zugeführt. Insbesondere falls das in dem Teilchen 1 enthaltene Pharmakon 9 lediglich ein eiziges Partikel ist, kann die Membran 3 derart beschaffen sein, dass sich diese mit der Freigabe des Pharmakons 9 zerstört.

Die Fig. 4 zeigt ein Teilchen 1 mit einem Reaktionsbereich 12 innerhalb des Partikelkerns 2. Abweichend von der Darstellung kann auch der gesamte Partikelkern 2 aus dem Reaktionsbereich 12 gebildet sein. Damit kann ein sogenanntes Metabolisierungs-Kompartiment gebildet sein. Die Membran 3 weist im Ausführungsbeispiel zwei Funktionselemente auf, welche als Einschleusungselement 5a und als Ausschleusungselement 5b zur Internalisierung bzw. Externalisierung eines Stoffes in das bzw. aus dem Teilchen 1 vorgesehen sind. Die Funktionselemente 5 haben jeweils kombinierte Detektor- und Transportfunktionen. Das Einschleusungselement 5a dient der Aufnahme eines körpereigenen Zwischenstoffes 13 in den Reaktionsbereich 12. Der körpereigene Zwischenstoff 13 ist ein vom Organismus des Patienten selbst hergestellter Stoff, welcher im Organismus weiter zu einem Endstoff 14 umzuwandeln ist. Diese körpereigene Umwandlung sei gestört. Die entsprechende Funktion wird vom Reaktionsbereich 12 mit Hilfe eines Reaktionsstoffes 15 übernommen. In der symbolischen Skizze des Ausführungsbeispiels befindet sich der Reaktionsstoff 15 zunächst außerhalb des Reaktionsbereiches 12 und tritt in diesen erst ausgelöst durch die Anwesenheit des Zwischenstoffes 13 im Partikelkern 2 ein. Abweichend von dieser symbolischen Darstellung kann der Zwischenstoff 13 beispielsweise auch den Reaktionsbereich 12 oder den Partikelkern 2 teilweise oder vollständig bilden. Das Teilchen 1 kann zur einfachen oder mehrfach aufeinanderfolgenden Umwandlung eines körpereigenen Zwischenstoffes 13 beziehungsweise körpereigener Zwischenstoffes 13 in einen Endstoff 14 vorgesehen sein. Im erstgenannten Fall kann die Membran 3 derart beschaffen sein, dass sich diese mit der Bildung des Endstoffes 14 auflöst. Der Zwischenstoff 13 kann auch zumindest einen Teil der Membran 3 bilden. Insgesamt bildet das Teilchen 1 eine Nanofabrik, etwa wie ein künstliches Ribosom, welche ausschließlich bestimmte Reaktionsprodukte, insbesondere Wirkstoffe, produziert und freisetzt.

Insbesondere das Teilchen 1 mit von der Membran 3 eingeschlossenem Reaktionsbereich 12, wie im Ausführungsbeispiel nach Fig. 4 symbolisiert, eignet sich zur gewebeselektiven Verabreichung von Genarznei. Die Genarznei als Pharmakon 9 kann dabei erst im Zielgewebe, ausgelöst durch die jeweiligen Reaktionsbedingungen und/oder durch gezielte externe Einflüsse, erzeugt werden. Ebenso besteht jedoch auch die Möglichkeit, ein Diagnostikum ausschließlich oder überwiegend in einem Zielgewebe freizusetzen.

Die Fig. 5 zeigt ein Ausführungsbeispiel, in welchem ein Teilchen 1 physikalisch und/oder datentechnisch mit externen Systemen verbindbar ist. Der Partikelkern 2 enthält, ähnlich wie beim Ausführungsbeispiel nach Fig. 3, ein Pharmakon 9, welches durch ein Funktionselement 5 hindurch aus der Membran 3 ausschleusbar ist. Die Membran 3 weist darüber hinaus ein Empfängerelement 16 auf, welches durch ein externes Signal 17, beispielsweise ein Ultraschallsignal oder ein elektromagnetisches Signal ansteuerbar ist. Die Ansteuerung der von außen beeinflußbaren Membran 3 geschieht unter Verwendung einer sogenannten Membran Exchange Software (MES). Im Fall der Ansteuerung durch ein elektromagnetisches Signal sollte die Ausdehnung des Empfängerelementes 16 ungefähr in der Größenordnung oder etwas unterhalb der Wellenlänge des externen Signals 17 sein. Wird beispielsweise Licht, insbesondere Infrarotlicht, als externes Signal 17 verwendet, so ist diese Anforderung mit einem Empfängerelement 16 im oder unterhalb des Mikrometer-Bereichs realisierbar. Das Empfängerelement 16 ist funktional, in der Darstellung durch Pfeile angedeutet, mit dem Schleusenelement 5 gekoppelt. Abweichend von der Darstellung ist es auch möglich, das Detektor- oder Empfängerelement 16 mit dem Schleusenelement in einem einzigen Funktionselement 5 zu vereinen. Durch die Einwirkung des externen Signals 17 kann in diesem Fall beispielsweise das Schleusenelement 5 aufgebrochen werden, so dass es - wie im Ausführungsbeispiel nach Fig. 5 - einen Teilchentransport von innen nach außen oder - wie im Ausführungsbeispiels nach Fig. 1 - einen Teilchentransport von außen nach innen ermöglicht. Besonders vorteilhaft ist die Möglichkeit, einen extern ausgelösten Teilchentransport durch die Membran 3 mit bildgebenden medizintechnischen Verfahren zu kombinieren.

## Patentansprüche

1. In einer Fluidbahn, insbesondere einer Blutbahn, transportierbares Teilchen mit einer einen Partikelkern (2) einschließenden Membran (3) mit einer Anzahl in einer Matrix (4) eingebundener Funktionselemente (5,5a,5b,8,10,16), welche in Abhängigkeit von der Konzentration eines Körperstoffes (7,11) einen Stofftransport durch die und/oder eine Stoffanlagerung an der Membran (3) bewirken, **dadurch gekennzeichnet, dass** als Funktionselement ein Detektorelement (10, 16) vorgesehen ist, wobei die Stoffanlagerung an der und/oder der Stoffdurchtritt durch die Membran (3) durch ein zur Ansteuerung des Detektorelementes (10, 16) vorgesehenes extrakorporales Signal (17) beeinflussbar ist.

2. Teilchen nach Anspruch 1, **dadurch gekennzeichnet, dass** dieses in einer Blutbahn eines Menschen oder Tieres transportierbar ist.

3. Teilchen nach Anspruch 1 oder 2, **dadurch gekennzeichnet, dass** die Membran (3) einen körpereigenen Stoff (7,11) akkumuliert.

4. Teilchen nach einem der Ansprüche 1 bis 3, **dadurch gekennzeichnet, dass** von der Konzentration des Körperstoffes (7,11) die Abgabe eines Pharmakons (9) durch die Membran (3) abhängig ist.

5. Teilchen nach einem der Ansprüche 1 bis 4, **dadurch gekennzeichnet, dass** die Membran (3) im Körper enzymatisch angreifbar ist.

6. Teilchen nach einem der Ansprüche 1 bis 5, **dadurch gekennzeichnet, dass** der Partikelkern (2) einen zur Stoffumwandlung vorgesehenen Reaktionsbereich (12) umfasst.

7. Teilchen nach Anspruch 6, **dadurch gekennzeichnet, dass** der Reaktionsbereich (12) zur Umwandlung eines körpereigenen Zwischenstoffes (13) vorgesehen ist.

8. Teilchen nach einem der Ansprüche 1 bis 7, **dadurch gekennzeichnet, dass** der Außendurchmesser (D) mindestens 50 nm und maximal 10 µm beträgt.

9. Teilchen nach einem der Ansprüche 1 bis 8, **dadurch gekennzeichnet, dass** die Dicke (d) der Membran (3) mindestens 2 nm und maximal 1 µm beträgt.

10. Teilchen nach einem der Ansprüche 1 bis 9, **dadurch gekennzeichnet, dass** das extrakorporale Signal (17) ein Ultraschallsignal ist.

11. Teilchen nach Anspruch 10, **dadurch gekennzeichnet, dass** das extrakorporale Signal (17) ein elektromagnetisches Signal ist.

12. Teilchen nach einem der Ansprüche 1 bis 11, **dadurch gekennzeichnet, dass** als Funktionselement ein einen Stofftransport durch die Matrix (4) ermöglichendes Schleusenelement (5,5a,5b) vorgesehen ist.

13. Teilchen nach Anspruch 12, **dadurch gekennzeichnet, dass** das Detektorelement (10,16) funktional mit dem Schleusenelement (5,5a,5b) gekoppelt ist.

14. Teilchen nach einem der Ansprüche 1 bis 13, **dadurch gekennzeichnet, dass** die Matrix (4) aus einer Polymerschicht gebildet ist.

15. Verfahren zur Detektion eines Teilchens nach einem der Ansprüche 1 bis 14, wobei die durch den Stofftransport durch die und/oder die Stoffanlagerung an der Membran (3) bewirkte Änderung des Teilchens (1) in einem bildgebenden medizintechnischen Verfahren detektiert wird.
